# EUROPEAN PATENT SPECIFICATION

(11) **EP 1 561 488 B1**
(45) Date of publication and mention of the grant of the patent: **14.09.2016**
(21) Application number: 05001850.6
(22) Date of filing: 28.01.2005
(51) Int. Cl.: A61M 5/145

(54) **Device for locking syringes to infusion pumps**
Vorrichtung zum Verriegeln der Spritzen zu Infusionspumpen
Dispositif de verrouillage des seringues aux pompes d'infusion

(30) Priority: 06.02.2004 IT MO20040027
(43) Date of publication of application: 10.08.2005
(73) Proprietor: SIDAM S.r.l., 41037 Mirandola-Frazione San Giacomo Roncole (MO) (IT)
(72) Inventor: Azzolini, Graziano, 41032 Cavezzo (Prov. of Modena) (IT)
(74) Representative: Brunacci, Marco

(56) References cited:
- WO-A-01/08727
- US-A- 4 465 475
- US-A- 4 608 042
- US-A- 4 908 017

## Description

The present invention relates to a device for locking syringes to infusion pumps.

Infusion pumps for syringes are known which are used in the therapeutic or diagnostic field for the controlled and automated infusion of drugs, contrast media or other fluids.

Known pumps are substantially constituted by a supporting frame, with which means for locking the cylindrical body of a syringe and means of the linear automated type for actuating the sliding of the plunger of the syringe are associated.

An electronic circuit controls and actuates the actuation means in order to dispense the fluid contained in the syringe according to an infusion program (time, flow-rate, speed et cetera) that can be set from the outside by an operator by means of a keypad and a display.

Conventional locking means comprise a block, which is rigidly coupled to the pump frame and in which there is a slot in which it is possible to insert the wings formed at the end of the cylindrical body from which the plunger protrudes, and a clamp, which wraps around the cylindrical body in an upper region; the block prevents the axial sliding of the cylindrical body and the clamp prevents its rotation.

These conventional locking means are not free from drawbacks, including the fact that they are structurally and constructively very complicated, scarcely versatile and adaptable, and in particular do not allow oscillation of the syringe.

Other drawbacks of known locking means consist in that they cause operations for coupling and uncoupling the syringes to be awkward to perform on the part of health workers and in that they cannot be used and adapted easily to lock syringes to the rotating shafts with which certain infusion pumps are provided in order to make said syringes oscillate so as to mix the fluid contained therein.

From patent document WO 01/08727 A1 it is known an adapter which is suitable for releasably attaching a syringe to a powered injector, wherein this adapter comprises a syringe carrier, that is adapted to seat at least a portion of the syringe; a releasable mounting mechanism, positioned to the rear of the syringe carrier, to mount the adapter to the front wall of the injector; and a first and a second section, that are rotatable relative to each other about a hinge axis generally perpendicular to a longitudinal axis of the adapter, with a drive member of the injector being provided for driving the axial movement of a plunger of the syringe.

However even this adapter and in general the system, of which the adapter is a part, for controlling the dispensing of a liquid solution from the syringe appear to request further improvements, in which for instance the practicality and facility, as allowed by this adapter, for attaching the syringe to the injector is associated with additional performances, namely for handling and mixing the liquid contained in the syringe, before and while being dispensed to a patient.

The aim of the present invention is to eliminate the drawbacks noted above of known locking means, by providing a device for locking syringes to infusion pumps that is structurally and constructively simple, is versatile in use and adaptable to the various dimensions of the syringes that can be used, allows to facilitate and simplify the operations performed by health workers to couple and disengage the syringes, and can be used easily to fix the syringes to rotating shafts with which the infusion pumps can be provided.

Within this aim, an object of the present invention is to provide a device that is simple, relatively easy to provide in practice, safe in use, effective in operation, and has a relatively low cost.

This aim and this and other objects that will become better apparent hereinafter are achieved by the present device for locking syringes to infusion pumps having the features of independent claim 1.

Further characteristics and advantages of the present invention will become better apparent from the following detailed description of a preferred but not exclusive embodiment of a device for locking syringes to infusion pumps, illustrated by way of non-limiting example in the accompanying drawings, wherein:

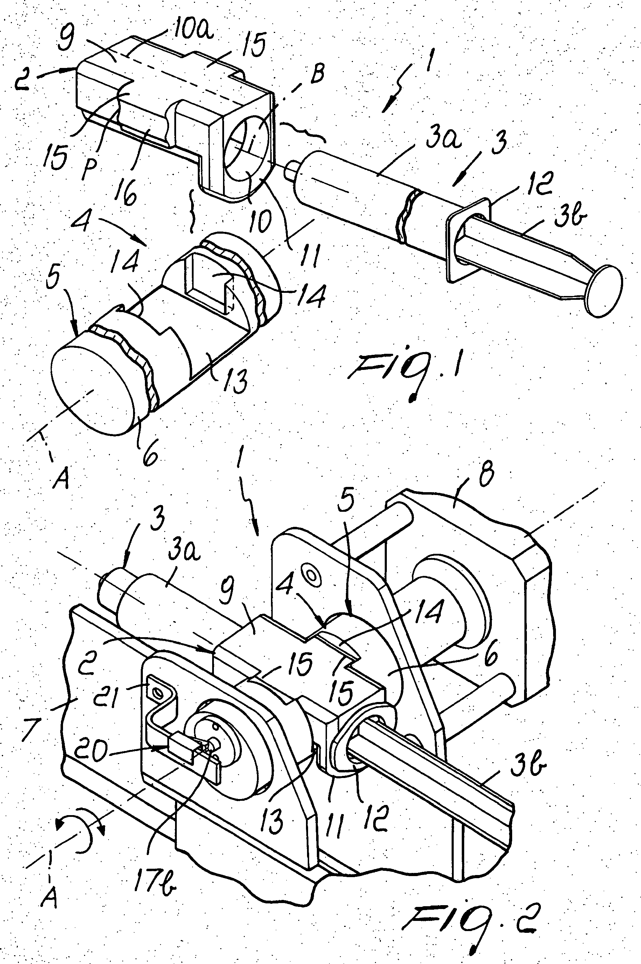

Figure 1 is an exploded schematic perspective view of a device for locking syringes to infusion pumps according to the invention;
Figure 2 is a schematic perspective view of the device of Figure 1, applied to an infusion pump;

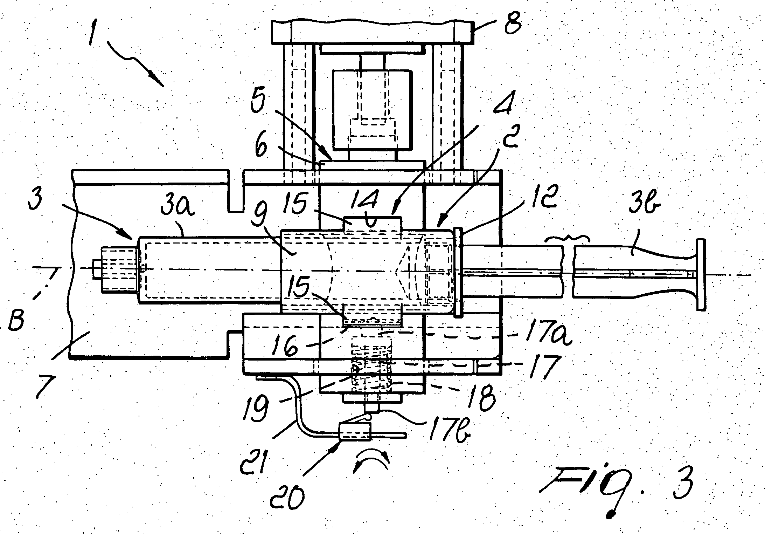

Figure 3 is a schematic top plan view of the device of Figure 2.

With reference to the figures, the reference numeral 1 generally designates a device for locking syringes to infusion pumps, which are generally not shown as they are of a known type.

The device 1 comprises supporting means 2 for supporting a syringe 3, of the type that comprises a substantially cylindrical body 3a and inside which a plunger 3b is inserted so that it can slide axially, said supporting means being rigidly associable with the body 3a, and coupling means 4 for temporarily coupling the supporting means 2 to an anchoring element 5 formed in a pump.

The anchoring element 5 can be constituted for example by a shaft 6, which is provided with a longitudinal axis A that is substantially perpendicular to a longitudinal axis B of the syringe 3, is arranged substantially horizontally, and is supported so that it can rotate about its own longitudinal axis A by a frame 7 of the pump.

The shaft 6 is associated with motor means 8, which are suitable to rotate it alternately through a preset angle; the syringe 3, which is anchored to the shaft 6, is thus made to oscillate in order to mix the components of the fluid to be infused that are contained therein.

The supporting means 2 comprise a substantially tubular element 9, in an internal cavity 10 of which it is possible to insert the body 3a, fixing means for example of the interlocking, adhesive or interference-coupling type for fixing the element 9 to the body 3a being provided.

In one possible embodiment, the fixing means comprise a longitudinal slit 10a that is formed in the element 9, the inside diameter of which, in the configuration for disengagement from the body 3a, is substantially smaller than the outside diameter of the body 3 a.

A substantially annular surface 11 for the abutment of wings or flange 12, formed proximate to the end of the body 3a in which the plunger 3b is inserted, is formed at one end of the element 9.

The coupling means 4 can be of the interlocking and/or barrier and/or friction types.

In the illustrated embodiment, the coupling means 4 comprise a receptacle 13 for containing the supporting means 2, which is formed in the anchoring element 5 and has a profile, i.e. a peripheral shape, that substantially duplicates or corresponds to the profile, i.e. the peripheral shape, of a corresponding portion of the outer lateral surface of the supporting means 2.

Cavities 14 are formed in the receptacle 13, and corresponding protrusions 15 formed in the supporting means 2 can be inserted therein; the cavities 14 and the protrusions 15 are prism-shaped.

Moreover, the supporting means 2 comprise a portion 16, which has a contoured profile P and is suitable to mate with a likewise contoured head 17a of a stem 17 that is inserted so that it can slide axially, with the interposition of elastic means 18, in a guide 19 that is formed in the anchoring element 5 at the receptacle 13; the head 17a protrudes within the receptacle 13 and the coupling between said head and the portion 16, together with the reaction of the elastic means 18, is suitable to fix the supporting means 2 to the anchoring element 5.

Means 20 for sensing presence of the syringe 3, such as on-off switches, sensors or the like, are further provided and are associated with the anchoring element 5; in particular, the sensing means 20 are fixed to a plate 21, which is anchored to the frame 7 proximate to an end of the anchoring element 5 at which there is an opening, from which the end 17b of the stem 17 that lies opposite the head 17a protrudes.

The stem 17 is suitable to activate the sensing means 20; by coupling the supporting means 2, to which the syringe 3 is fixed, to the cavity 13, their portion 16 mates with the head 17a of the stem 17, causing it to slide toward the outside of the anchoring element 5, therefore causing the activation of the sensing means 20, which report to a central control and monitoring unit the presence of the supporting means 2, i.e., of the syringe 3 that is rigidly coupled thereto.

In practice it has been found that the described invention achieves the intended aim and object.

The locking device according to the invention is in fact structurally and constructively simple and versatile and can be adapted to syringes of various sizes; it allows to facilitate the operations for engaging and disengaging the syringes that are performed by health workers and it can be used easily to fix syringes to the rotating shafts with which infusion pumps may be provided.

The invention thus conceived is susceptible of numerous modifications and variations, all of which are within the scope of the appended claims.

All the details may further be replaced with other technically equivalent ones.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An infusion pump with a device for locking syringes, comprising:
- an infusion pump provided with a frame (7) and with an anchoring element (5);
- supporting means (2) for supporting a syringe (3) of the type that comprises a substantially cylindrical body (3a) inside which a plunger (3b) is inserted so that it can slide axially, with said supporting means (2) being rigidly associable with said substantially cylindrical body (3a),
**characterized in that** it further comprises coupling means (4) for temporarily fixing said supporting means (2) to said anchoring element (5) of the infusion pump and **in that** said anchoring element (5) is constituted by a shaft (6), which extends along a longitudinal axis (A) thereof that is substantially at right angles to the longitudinal axis (B) of said syringe (3) and is supported, so as to perform rotation about said longitudinal axis (A) thereof, by said frame (7).

2. The pump according to claim 1, **characterized in that** it comprises motor means (8) associated to said shaft (6) which are suitable to rotate it alternately through a preset angle.

3. The pump according to claim 1 or 2, **characterized in that** said supporting means (2) comprises a substantially tubular element (9), in which it is possible to insert said substantially cylindrical body (3a), and fixing means (10a) for rigidly fixing said substantially tubular element (9) to said substantially cylindrical body (3a).

4. The pump according to claim 3, **characterized in that** said fixing means are of the interlocking, adhesive-bonding or interference-coupling type.

5. The pump according to claim 3 or 4, **characterized in that** said fixing means comprise a longitudinal slit (10a), which is formed in said substantially tubular element (9), with the inside diameter of said substantially tubular element (9) being substantially smaller than the outside diameter of said substantially cylindrical body (3a).

6. The pump according to claim 3 or 4 or 5, **characterized in that** said substantially tubular element (9) comprises, at one end, a substantially annular surface (11) for the abutment of the wings (12) formed proximate to the end of said substantially cylindrical body (3a) in which said plunger (3b) is inserted.

7. The pump according to one or more of the preceding claims, **characterized in that** said temporary coupling means (4) are of the interlocking and/or barrier and/or friction type.

8. The pump according to one or more of the preceding claims, **characterized in that** said temporary coupling means (4) comprise a receptacle (13) for containing said supporting means (2), with said receptacle (13) being formed in said anchoring element (5).

9. The pump according to claim 8, **characterized in that** said containment receptacle (13) has a profile that substantially duplicates the profile of a corresponding portion of the outer lateral surface of said supporting means (2).

10. The pump according to claim 8 or 9, **characterized in that** said containment receptacle (13) is provided with cavities (14) in which it is possible to insert corresponding protrusions (15) formed at said supporting means (2).

11. The pump according to claim 10, **characterized in that** said cavities (14) and said protrusion (15) are prism-shaped.

12. The pump according to one or more of the preceding claims, **characterized in that** said supporting means (2) comprises a portion (16) that has a contoured profile (P) and is suitable to mate with the head (17a) of a stem (17) that is inserted so that it can slide axially, with the interposition of elastic means (18), in a guide (19) that is formed in said anchoring element (5) at said containment receptacle (13), said head (17a) protruding within said containment receptacle (13), the coupling between said head (17a) and said portion (16) having a contoured profile being suitable to fix said supporting means (2) to said anchoring element (5).

13. The pump according to one or more of the preceding claims, **characterized in that** it comprises sensing means (20) for sensing presence of said syringe (3), such as on/off switches, sensors or the like, which are associated with said anchoring element (5).

14. The pump according to claim 13 as dependent on claim 12, **characterized in that** the end (17b) of said stem (17) that lies opposite said head (17a) protrudes externally from said anchoring element (5), with said sensing means (20) being arranged proximate to said end (17b), and said stem (17) being suitable to actuate said sensing means (20).

## Patentansprüche

1. Infusionspumpe mit einer Vorrichtung zum verriegeln von Spritzen, umfassend:
- eine Infusionspumpe, die mit einem Rahmen (7) und mit einem Verankerungselement (5) versehen ist;
- Haltemittel (2) zum Halten einer Spritze (3) des Typs, der einen im Wesentlichen zylindrischen Körper (3a) umfasst, in dem ein Kolben (3b) eingesetzt ist, so dass dieser axial gleiten kann, wobei das Haltemittel (2) starr mit dem im Wesentlichen zylindrischen Körper (3a) zusammenfügbar ist,
**dadurch gekennzeichnet, dass** sie des Weiteren Kopplungsmittel (4) zum temporären Befestigen des Haltemittels (2) an dem Verankerungselement (5) der Infusionspumpe umfasst und dass das Verankerungselement (5) durch eine Welle (6) gebildet ist, welche sich entlang einer Längsachse (A) davon erstreckt, die im Wesentlichen im rechten Winkel zu der Längsachse (B) der Spritze (3) steht und durch den Rahmen (7) gehalten wird, um eine Drehung um die Längsachse (A) davon auszuführen.

2. Pumpe nach Anspruch 1, **dadurch gekennzeichnet, dass** sie Motormittel (8) umfasst, die der Welle (6) zugeordnet sind, welche geeignet sind, sie um einen voreingestellten Winkel abwechselnd zu drehen.

3. Pumpe nach Anspruch 1 oder 2, **dadurch gekennzeichnet, dass** das Haltemittel (2) ein im Wesentlichen rohrförmiges Element (9) umfasst, bei dem es möglich ist, den im Wesentlichen zylindrischen Körper (3a) einzusetzen, und Befestigungsmittel (10a) umfasst, um das im Wesentlichen rohrförmige Element (9) an dem im Wesentlichen zylindrischen Körper (3a) starr zu befestigen.

4. Pumpe nach Anspruch 3, **dadurch gekennzeichnet, dass** die Befestigungsmittel vom verriegelnden, klebendverbindenden oder presspassendem Typ sind.

5. Pumpe nach Anspruch 3 oder 4, **dadurch gekennzeichnet, dass** die Befestigungsmittel einen Längsschlitz (10a) umfassen, der in dem im wesentlichen rohrförmige Element (9) ausgebildet ist, wobei der Innendurchmesser des im Wesentlichen rohrförmigen Elements (9) im Wesentlichen kleiner ist als der Außendurchmesser des im Wesentlichen zylindrischen Körpers (3a).

6. Pumpe nach Anspruch 3 oder 4 oder 5, **dadurch gekennzeichnet, dass** das im Wesentlichen rohrförmige Element (9) an einem Ende eine im Wesentlichen ringförmige Fläche (11) für das Widerlager der Flügel (12) umfasst, die nahe dem Ende des im Wesentlichen zylindrischen Körpers (3a) ausgebildet sind, in den der Kolben (3b) eingesetzt ist.

7. Pumpe nach einem oder mehreren der vorhergehenden Ansprüche, **dadurch gekennzeichnet, dass** die temporären Kupplungsmittel (4) vom Verriegelungs- und/oder Sperr- und/oder Reibungstyp sind.

8. Pumpe nach einem oder mehreren der vorhergehenden Ansprüche, **dadurch gekennzeichnet, dass** die temporären Kupplungsmittel (4) ein Buchsenteil (13) zum Aufnehmen des Haltemittels (2) umfassen, wobei das Buchsenteil (13) im Verankerungselement (5) ausgebildet ist.

9. Die Pumpe nach Anspruch 8, **dadurch gekennzeichnet, dass** das Aufnahmebuchsenteil (13) ein Profil aufweist, das im Wesentlichen das Profil eines entsprechenden Abschnitts der äußeren Fläche des Haltemittels (2) dupliziert.

10. Pumpe nach Anspruch 8 oder 9, **dadurch gekennzeichnet, dass** das Aufnahmebuchsenteil (13) mit Kavitäten (14) versehen ist, in die das Einsetzen entsprechender Vorsprünge (15), die an dem Haltemittel (2) ausgebildet sind, möglich ist.

11. Pumpe nach Anspruch 10, **dadurch gekennzeichnet, dass** die Kavitäten (14) und der Vorsprung (15) prismenförmig sind.

12. Pumpe nach einem oder mehreren der vorhergehenden Ansprüche, **dadurch gekennzeichnet, dass** das Haltemittel (2) einen Abschnitt (16) umfasst, der ein konturiertes Profil (F) aufweist und geeignet ist, mit dem Kopf (17a) eines Schafts (17) zusammenzupassen, der eingesetzt wird, so dass er axial unter Zwischenschaltung von elastischen Mitteln (18) in einer Führung (19) gleiten kann, welche im Verankerungselement (5) an dem Aufnahmebuchsenteil (13) ausgebildet ist, wobei der Kopf (17a) innerhalb des Aufnahmebuchsenteils (13) vorragt, wobei die Kopplung zwischen dem Kopf (17a) und dem Abschnitt (16) mit einem konturierten Profil geeignet ist, das Haltemittel (2) an dem Verankerungselement (5) zu befestigen.

13. Pumpe nach einem oder mehreren der vorhergehenden Ansprüche, **dadurch gekennzeichnet, dass** sie Sensormittel (20) zum Erfassen des Vorhandenseins der Spritze (3), wie beispielsweise Ein/Aus-Schalter, Sensoren oder dergleichen, umfasst, die mit dem Verankerungselement (5) verbunden sind.

14. Pumpe nach Anspruch 13, sofern abhängig von Anspruch 12, **dadurch gekennzeichnet, dass** das Ende (17b) des Schaftes (17), das gegenüber dem Kopf (17a) liegt, nach außen aus dem Verankerungselement (5) vorragt, wobei das Sensormittel (20) nahe dem Ende (17b) angeordnet ist und wobei der Schaft (17) geeignet ist, das Sensormittel (20) zu betätigen.

## Revendications

1. Pompe à perfusion avec un dispositif de verrouillage de seringues, comprenant:
- une pompe à perfusion pourvue d'une armature (7) et d'un élément d'ancrage (5) ;
- des moyens de support (2) destinés à porter une seringue (3) du type qui comprend un corps sensiblement cylindrique (3a) à l'intérieur duquel un piston plongeur (3b) est inséré de telle sorte qu'il peut coulisser axialement, lesdits moyens de support (2) pouvant être associés de manière rigide audit corps sensiblement cylindrique (3a),
**caractérisée en ce qu'**elle comprend en outre des moyens de couplage (4) destinés à fixer temporairement lesdits moyens de support (2) audit élément d'ancrage (5) de la pompe à perfusion, et **en ce que** ledit élément d'ancrage (5) est constitué par un arbre (6) qui s'étend le long d'un axe longitudinal (A) de celui-ci, qui est sensiblement à angle droit par rapport à l'axe longitudinal (B) de ladite seringue (3), et qui est porté par ladite armature (7) de manière à être en rotation autour dudit axe longitudinal (A) de l'élément.

2. Pompe selon la revendication 1, **caractérisée en ce qu'**elle comprend des moyens moteurs (8) associés audit arbre (6) et qui sont aptes à le mettre en rotation de manière alternée vers un angle prédéterminé.

3. Pompe selon la revendication 1 ou 2, **caractérisée en ce que** lesdites moyens de support (2) comprennent un élément sensiblement tubulaire (9), dans lequel il est possible d'insérer ledit corps sensiblement cylindrique (3a), et des moyens de fixation (10a) destinés à fixer de manière rigide ledit élément sensiblement tubulaire (9) audit corps sensiblement cylindrique (3a).

4. Pompe selon la revendication 3, **caractérisée en ce que** lesdits moyens de fixation sont du type à emboîtement, à liaison par collage, ou à couplage par interférence.

5. Pompe selon la revendication 3 ou 4, **caractérisée en ce que** lesdits moyens de fixation comprennent une fente longitudinale (10a), qui est formée dans ledit élément sensiblement tubulaire (9), le diamètre intérieur dudit élément sensiblement tubulaire (9) étant sensiblement inférieur au diamètre extérieur dudit corps sensiblement cylindrique (3a).

6. Pompe selon la revendication 3 ou 4 ou 5, **caractérisée en ce que** ledit élément sensiblement tubulaire (9) comprend, à une extrémité, une surface sensiblement annulaire (11) pour la venue en butée des ailes (12) formées au voisinage de l'extrémité dudit corps sensiblement cylindrique (3a) dans lequel ledit piston plongeur (3b) est inséré.

7. Pompe selon une ou plusieurs des revendication précédentes, **caractérisée en ce que** lesdits moyens de couplage temporaire (4) sont du type à emboîtement et/ou à barrière et/ou à friction.

8. Pompe selon une ou plusieurs des revendications précédentes, **caractérisée en ce que** lesdits moyens de couplage temporaire (4) comprennent un réceptacle (13) destiné au confinement desdits moyens de support (2), ledit réceptacle (13) étant formé dans ledit élément d'ancrage (5).

9. Pompe selon la revendication 8, **caractérisée en ce que** ledit réceptacle de confinememt (13) a un profil qui reproduit sensiblement le profil d'une partie correspondante de la surface latérale extérieure desdits moyens de support (2).

10. Pompe selon la revendication 8 ou 9, **caractérisée en ce que** ledit réceptacle de confinememt (13) est pourvu de cavités (14) dans lesquelles il est possible d'insérer des éléments en saillie (15) correspondants, formés au niveau desdits moyens de support (2).

11. Pompe selon la revendication 10, **caractérisée en ce que** lesdites cavités (14) et lesdits éléments en saillie (15) sont en forme de prisme.

12. Pompe selon une ou plusieurs des revendications précédentes, **caractérisée en ce que** lesdits moyens de support (2) comprennent une partie (16) qui présente un profil (P) avec un certain contour et qui est apte à coopérer avec la tête (17a) d'une tige (17) qui est insérée de manière à ce qu'elle puisse coulisser axialement - avec interposition de moyens élastiques (18) - dans un guide (19) qui est formé dans ledit élément d'ancrage (5) au niveau dudit réceptacle de confinememt (13), ladite tête (17a) étant en saillie à l'intérieur dudit réceptacle de confinememt (13), le couplage entre ladite tête (17a) et ladite partie (16) ayant un profil avec un certain contour qui est apte à fixer lesdits moyens de support (2) audit élément d'ancrage (5).

13. Pompe selon une ou plusieurs des revendications précédentes, **caractérisée en ce qu'**elle comprend des moyens de détection (20) destinés à détecter la présence de ladite seringue (3), tels que des interrupteurs marche/arrêt, des capteurs ou autres du même type, et qui sont associés audit élément d'ancrage (5).

14. Pompe selon la revendication 13 dans la mesure où celle-ci dépend de la revendication 12, **caractérisée en ce que** l'extrémité (17b) de ladite tige (17) qui est située à l'opposé de ladite tête (17a) est en saillie à l'extérieur dudit élément d'ancrage (5), lesdits moyens de détection (20) étant disposés au voisinage de ladite extrémité (17b), et ladite tige (17) étant apte à actionner lesdits moyens de détection (20).
